(19) Europäisches Patentamt
European Patent Office
Office européen des brevets

(11) Veröffentlichungsnummer : **0 006 971**
**B1**

(12) # EUROPÄISCHE PATENTSCHRIFT

(45) Veröffentlichungstag der Patentschrift : **02.12.81**

(51) Int. Cl.³ : **C 07 D285/12**, A 61 K 31/41// C07D417/12

(21) Anmeldenummer : **79100996.2**

(22) Anmeldetag : **31.03.79**

(54) **Aminoderivate des 2-Methyl-5-(2-hydroxystyryl)-1,3,4-thiadiazols, Verfahren zu ihrer Herstellung und diese enthaltende therapeutische Mittel.**

(30) Priorität : **28.04.78 DE 2818765**

(43) Veröffentlichungstag der Anmeldung : **23.01.80 (Patentblatt 80/02)**

(45) Bekanntmachung des Hinweises auf die Patenterteilung : **02.12.81 Patentblatt 81/48**

(84) Benannte Vertragsstaaten : **BE CH DE FR GB IT LU NL SE**

(56) Entgegenhaltungen : **DE - A - 2 216 537**

(73) Patentinhaber : **BASF Aktiengesellschaft**
**Carl-Bosch-Strasse 38**
**D-6700 Ludwigshafen (DE)**

(72) Erfinder : **Thieme, Peter C., Dr.**
**Dr.-Hans-Hoffmann-Strasse 5**
**D-6706 Wachenheim (DE)**
Erfinder : **Frickel, Fritz-Frieder, Dr.**
**Prager Strasse 27**
**D-6700 Ludwigshafen (DE)**
Erfinder : **Hagen, Helmut, Dr.**
**Max-Slevogt-Strasse 17 e**
**D-6710 Frankenthal (DE)**
Erfinder : **Franke, Albrecht, Dr.**
**Mandelring 11**
**D-6706 Wachenheim (DE)**
Erfinder : **Lenke, Dieter, Dr.**
**Kekuleplatz 1**
**D-6700 Ludwigshafen (DE)**
Erfinder : **Gries, Josef, Dr.**
**Roemerweg 43**
**D-6706 Wachenheim (DE)**

**EP 0 006 971 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Aminoderivate des 2-Methyl-5-(2-hydroxystyryl)-1,3,4-thiadiazols, Verfahren zu ihrer Herstellung und diese enthaltende therapeutische Mittel

Die vorliegende Erfindung betrifft neue Alkylaminopropanolderivate des 2-Methyl-5-(2-hydroxystyryl)-1,3,4-thiadiazols und ihre Säureadditionssalze, ihre Herstellung und diese Verbindungen enthaltende pharmazeutischen Zubereitungen, die zur Behandlung der coronaren Herzkrankheit, zur Hypertonie und von Herzrhythmusstörungen verwendet werden können.

In der DE-OS 2 216 537 werden beispielsweise Alkylaminopropanolderivate der o-Hydroxyzimtsäure, die eine β-sympatholytische Wirkung aufweisen, beschrieben. Ihre Wirkungen im Hinblick auf Nebenwirkungen und therapeutische Breite befriedigen nicht immer.

Es wurde gefunden, daß Verbindungen der allgemeinen Formel (I),

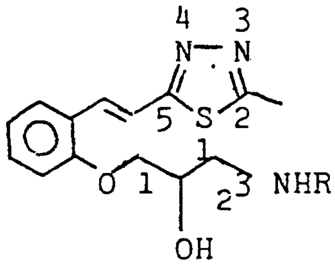

(I)

in denen R einen am zum Stickstoffatom α-ständigen Kohlenstoffatom verzweigten Alkylrest mit 3 bis 6 C-Atomen, einen Alkenyl- oder Alkinylrest mit je 2 bis 8 C-Atomen oder Cyclopropyl bedeutet, und ihre Säureadditionssalze wertvolle pharmakologische Eigenschaften aufweisen.

Alkylreste für R sind beispielsweise Isopropyl, tert.-Butyl, 2-Methyl-butyl-2, sek.-Butyl, 3-Methylpentyl-3 und Pentyl-2.

Als Alkenyl- oder Alkinylreste mit 2 bis 8 C-Atomen sind beispielsweise 1-Propen-3-yl, 3-Butin-2-yl, 2-Methyl-3-butin-2-yl und 3-Äthyl-1-pentin-3-yl zu nennen. Davon sind Alkinylreste mit 3 bis 6 C-Atomen, wie 3-Butin-2-yl und 2-Methyl-3-butin-2-yl, bevorzugt.

Die erfindungsgemäßen Verbindungen können hergestellt werden, indem man ein 2-Methyl-5-styryl-1,3,4-thiadiazol der allgemeinen Formel (II)

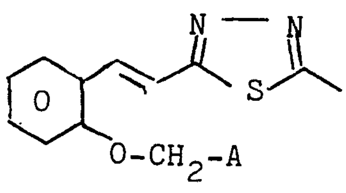

(II)

in der A den Rest

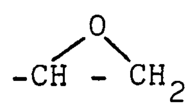

oder

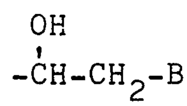

wobei B für eine nukleofuge Abgangsgruppe steht, bedeutet, mit einem Amin der allgemeinen Formel

$$H_2N—R$$

in der R die oben angegebenen Bedeutungen hat, zweckmäßigerweise in einem Lösungsmittel und ggf. in Gegenwart eines säurebindenden Mittels in an sich bekannter Weise umsetzt und die erhaltene Verbindung ggf. in das Säureadditionssalz einer physiologisch verträglichen Säure überführt.

Die Abgangsgruppe B stellt bevorzugt ein Halogenatom, insbesondere Chlor, Brom oder Jod, dar. Weiterhin kommen beispielsweise als nukleofuge Abgangsgruppen aromatische oder aliphatische Sulfonsäurereste in Betracht, wie der p-Toluolsulfonsäure-, der p-Brombenzolsulfonsäure- oder der Methansulfonsäurerest.

Die Umsetzungen werden bei Temperaturen von 10 bis 120 °C, d.h. bei Raumtemperaturen oder bei höheren Temperaturen, zweckmäßig bei Temperaturen von 50 bis 120 °C, durchgeführt. Die Umsetzungen können unter Atmosphärendruck oder in einem geschlossenen Gefäß unter erhöhtem Druck gegebenenfalls unter Erwärmung auf den angegebenen Temperaturbereich durchgeführt werden. Insbesondere bei leichtflüchtigen Aminen $H_2NR$ kann es vorteilhaft sein, die Umsetzung in einem

geschlossenen System, d.h. einem Autoklaven, durchzuführen.

Die Ausgangsverbindungen können direkt, d.h. ohne Zugabe eines Verdünnungs- oder Lösungsmittels, umgesetzt werden. Zweckmäßigerweise werden die Umsetzungen jedoch in Gegenwart eines inerten Verdünnungs- oder Lösungsmittels, beispielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, von Benzol oder eines Alkylbenzols, wie Toluol oder Xylol, oder eines aliphatischen Kohlenwasserstoffes, wie Hexan, Heptan oder Octan, eines niederen aliphatischen Ketons, wie Aceton, Methyläthylketon oder Methyl-isobutylketon, eines Dialkylformamids, wie Dimethyl- oder Diäthylformamid, von Dimethylsulfoxid oder in Gegenwart von Wasser oder in Mischungen der genannten Lösungsmittel, durchgeführt.

Auch ist das in überschüssiger Menge verwendete Amin der Formel $H_2N—R$ gegebenenfalls als Verdünnungs- oder Lösungsmittel geeignet.

Bevorzugte Lösungsmittel bei der Umsetzung von 2-Methyl-5-2-[(2,3-epoxypropoxy)-styryl]-1,3,4-thiadiazol mit einem Amin $R—NH_2$ sind niedere Alkohole, insbesondere Äthanol oder Isopropanol, wobei die Umsetzung bevorzugt bei Temperaturen von 50 °C bis 100 °C und bei Normaldruck durchgeführt wird.

Bei der nukleophilen Substitution eines Restes B sind als Lösungsmittel ein niederes aliphatisches Keton, wie Aceton, Methyläthylketon oder Methyl-isobutylketon, ein cyclischer Äther, insbesondere Tetrahydrofuran oder Dioxan, oder ein Dialkylformamid, wie Dimethylformamid, und Temperaturen von 90 bis 120 °C bevorzugt. Gegebenenfalls wird die Reaktion in Gegenwart einer katalytischen Menge Natrium- oder Kaliumjodid durchgeführt.

Es sei erwähnt, daß als Ausgangsverbindung der Formel (II) gegebenenfalls auch eine Mischung des Epoxids mit einem Halogenhydrin in Betracht kommt, da bei der technischen Herstellung der Ausgangsverbindungen der Formel II derartige Gemische unter Umständen entstehen können.

Bei einer zweckmäßigen Ausführungsform zur nukleophilen Substitution des Restes B durch das verwendete Amin wird die Umsetzung in Gegenwart einer Base als säurebindendes Mittel durchgeführt. Bevorzugte Basen sind Alkalimetallhydroxide, -carbonate, -hydrogencarbonate, -alkoholate oder ein tertiäres organisches Amin, wie Pyridin oder ein Trialkylamin, wie Trimethylamin oder Triäthylamin. Von den Alkaliverbindungen kommen insbesondere die des Natriums und Kaliums in Betracht. Dabei wird die Base in stöchiometrischer Menge oder in geringem Überschuß verwendet. Gegenbenenfalls ist es zweckmäßig, das zur Umsetzung verwendete Amin $H_2N—R$ in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z.B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, Überführen in eine Säureadditionsverbindung oder durch Säulenchromatographie.

Die Ausgangsverbindungen der Formel (II) können durch Alkylierung des 2-Methyl-5-(2-hydroxystyryl)-1,3,4-thiadiazols, das durch Kondensation von Salicylaldehyd mit 2,5-Dimethyl-1,3,4-thiadiazol, wie es im Beispiel I, Verbindung 1, beschrieben wird, hergestellt werden kann, mit einem Epihalogenhydrin oder einem α,ω-Dihalogen-2-propanol erhalten werden. Als Epihalogenhydrine kommen Epichlorhydrin, Epobromhydrin und Epijodhydrin und als α-ω-Dihalogen-2-propanol kommen insbesondere 1,3-Dichlor-2-propanol und 1,3-Dibrom-2-propanol in Betracht.

Die Umsetzungen des 2-Methyl-5-(2-hydroxystyryl)-1,3,4-thiadiazols zur Herstellung der Ausgangsverbindungen der Formel (II) werden zweckmäßigerweise bei Temperaturen von 0 bis 120 °C und unter Normaldruck oder in einem geschlossenen Gefäß unter erhöhten Drucken durchgeführt. Die Umsetzungen werden zweckmäßigerweise in einem inerten Verdünnungs- oder Lösungsmittel, z.B. einem niederen aliphatischen Keton, wie Aceton, Methyläthylketon oder Methylisobutylketon, einem niederen Alkohol mit 1 bis 4 C-Atomen, wie Methanol, Äthanol, Propanol oder Butanol, einem aliphatischen oder cyclischen Äther, wie Dialkyläther, Tetrahydrofuran oder Dioxan, einem Dialkylformamid, wie Dimethylformamid oder Diäthylformamid, oder Dimethylsulfoxid oder Hexamethylphosphortriamid oder mit überschüssigem Alkylierungsmittel als Verdünnungs- oder Lösungsmittel durchgeführt.

Bevorzugt werden die Umsetzungen in Gegenwart einer Base als säurebindendes Mittel vorgenommen. Geeignete Basen sind Alkalimetallcarbonate, -hydrogencarbonate, -hydroxide, -hydride oder -alkoholate, insbesondere des Natriums und Kaliums, basische Oxide, wie Aluminiumoxid oder Calciumoxid, organische tertiäre Basen, wie Pyridin, Piperidin oder niedere Trialkylamine, wie Trimethyl- oder Triäthylamin. Dabei können die Basen im Verhältnis zum eingesetzten Alkylierungsmittel in katalytischer Menge oder in stöchiometrischer Menge bzw. in geringem überschuß verwendet werden.

Bevorzugt wird das 2-Methyl-5-(2-hydroxystyryl)-1,3,4-thiadiazol mit Epibromhydrin oder 1,2-Dibrompropanol-2 in einem Lösungsmittelgemisch aus einem Äther und einem polaren aprotischen Lösungmittel, insbesondere Tetrahydrofuran und Hexamethylphosphortriamid, bei Temperaturen zwischen 0° und 50 °C umgesetzt.

Es wird darauf hingewiesen, daß das 2-Methyl-5-(2-hydroxystyryl)-1,3,4-thiadiazol Gegenstand der

europäischen Patentanmeldung 0 005 175 (Anmeldungs-Nr. 79101008.5) ist.

Die erfindungsgemäßen Verbindungen der Formel (I) können aufgrund der Kohlenstoff-Kohlenstoff-Doppelbindung als cis-trans-Isomerengemische auftreten. In der Regel werden jedoch nach dem üblichen physikalischen Reinigungsverfahren, wie fraktionierende Kristallisation, Chromatographie oder Sublimation, die trans-Isomeren erhalten.

Die erfindungsgemäßen Verbindungen der Formel (I) besitzen am Kohlenstoffatom 2 der aliphatischen Seitenkette ein Chiralitätszentrum und werden als Racemate erhalten, die durch bekannte Methoden, beispielsweise durch Bildung diastereomerer Salze mit optisch aktiven Hilfssäuren, wie Dibenzoylweinsäure, Campher-10-sulfonsäure, Ditoluylweinsäure oder 3-Brom-campher-8-sulfonsäure, in die optisch aktiven Antipoden getrennt werden können.

Gegebenenfalls werden die erhaltenen erfindungsgemäßen Verbindungen in das Säureadditionssalz einer physiologisch verträglichen Säure überführt. Als übliche physiologisch verträgliche organische oder anorganische Säuren kommen beispielsweise in Betracht Salzsäure, Bromwasserstoffsäure, Phosphorsäure oder Schwefelsäure, und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung, Band *10,* Seiten 224 bis 225, Birkhäuser Verlag, Basel und Stuttgart, 1966, oder dem Journal of Pharmaceuticals Sciences, Volume 66, Seiten 1 bis 5 (1977), entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Äthanol oder Propanol, oder einem niederen Keton, wie Aceton, Methyläthylketon oder Methylisobutylketon, oder einem Äther, wie Diäthyläther, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure-Additionsverbindungen der Aminopropanol-Derivate der allgemeinen Formel (I) durch Auflösen der freien Basen der allgemeinen Formel (I) in einer wäßrigen Säurelösung hergestellt werden.

Die erfindungsgemäßen Verbindungen und ihre physiologisch verträglichen Säureadditionssalze weisen wertvolle pharmakologische Eigenschaften auf.

Sie sind als Pharmaka mit beta-sympatholytischer Wirkung zur Behandlung der coronaren Herzkrankheit, der Hypertonie und von Herzrhythmusstörungen geeignet.

Die beta-sympatholytische Wirkung wurde an Katzen getestet. Als Vergleichssubstanz diente das bekannte Beta-Sympatholytikum Propranolol. Zur Prüfung wurden folgende Methoden verwendet:

1. Beta-sympatholytische Wirkung

Isoproterenol (1 µg/kg i.v.) verursacht an narkotisierten Katzen (Gewicht: 2 bis 4 kg) Herzfrequenzsteigerungen um durchschnittlich 40 %. Beta-Sympatholytica hemmen diese Tachycardien. Isoproterenol wurde vor, sowie 10 und 40 Minuten nach der i.v. Applikation der Prüfsubstanzen appliziert. Zwischen den Logarithmen der applizierten Dosen (mg/kg) der Prüfsubstanzen und der Hemmung der Isoproterenol-Tachycardie (%) bestehen lineare Beziehungen. Aus diesen Beziehungen werden als ED 50 % die Dosen ermittelt, welche die Isoproterenol-Tachycardie um 50 % hemmen.

2. Akute Toxizität

Zur Bestimmung der mittleren letalen Dosis (LD50) werden die Substanzen weiblichen NMRI-Mäusen (Gewicht: 19 bis 26 g) intraperitoneal appliziert.

Die in der Tabelle zusammengefaßten Ergebnisse zeigen, daß sich die erfindungsgemäßen Substanzen durch eine ungewöhnlich hohe beta-sympatholytische Aktivität auszeichnen. Die für die pharmakotherapeutische Anwendung bedeutsamen cardialen Beta-1-Rezeptoren werden bereits durch Dosen blockiert, die 12- (Beispiel 2) bis 25-mal (Beispiel 3) kleiner sind, als die der Vergleichssubstanz Propranolol. Aus dieser hohen Wirksamkeit und den letalen Dosen, die in der gleichen Größenordnung liegen wie die des Propranolols, ergibt sich eine Vergrößerung der therapeutischen Breite auf das 7,6- (Beispiel 2) bis 30,7-fache (Beispiel 7) des Propanolols.

Siehe die Tabelle Seite 5.

Tabelle: Beta-sympatholytische Wirkung und akute Toxizität

| Substanz | Beta-sympatholyt. W. (1) | | Akute Tox. | Therapeutische Breite (5) | |
|---|---|---|---|---|---|
| | ED 50 % (2) | R.W. (3) | LD 50 (4) | absolut | relativ (6) |
| Propranolol | 0,141 | 1,00 | 108 | 766 | 1,00 |
| Verbindung Beispiel 1 | 0,010 | 14,10 | 83,0 | 8 300 | 10,84 |
| Verbindung Beispiel 2 | 0,012 | 11,75 | 69,4 | 5 780 | 7,55 |
| Verbindung Beispiel 3 | 0,000 559 | 25,22 | 58,8 | 10 520 | 13,73 |
| Verbindung Beispiel 6 | 0,011 1 | 12,70 | 261 | 23 500 | 30,68 |

(1) Hemmung der Isoproterenol-Tachycardie. Katze. Hexobarbital-Narkose. Appl.: i.v.
(2) Dosis (mg/kg), welche die Isoproterenol-Tachycardie um 50 % hemmt.
(3) Relative Wirksamkeit. Propranolol = 1,00.
(4) Maus. Appl.: i.p.
(5) $\frac{\text{LD 50}}{\text{ED 50 \%}}$
(6) Propranolol = 1,00.

Gegenstand der vorliegenden Erfindung sind demnach auch therapeutische Mittel oder Zubereitungen, die neben üblichen Träger- und Verdünnungsmitteln eine Verbindung der Formel (I) als Wirkstoff enthalten, sowie die Verwendung der neuen Verbindungen zu therapeutischen Zwecken.

Die therapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen. Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Calciumcarbonat, Calciumphosphat oder Milchzucker, Sprengmitteln wie Maisstärke oder Alginsäure, Bindemitteln wie Stärke oder Gelatine, Gleitmitteln wie Magnesiumstearat oder Talk und/oder Mitteln zur Erzielung des Depoteffektes wie Carboxypolymethylen, Carboxymethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können. Lösungen oder Suspensionen mit den erfindungsgemäßen Wirkstoffen können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe, wie Natriumcarboxymethylcellulose, oder Konservierungsstoffe, wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln, wie Neutralfetten oder Polyäthylenglykol bzw. deren Derivaten, herstellen.

Als Einzeldosis der erfindungsgemäßen Verbindungen kommen am Menschen 1 bis 100 mg, vorzugsweise 2 bis 50 mg, in Betracht.

Bezüglich ihrer Wirksamkeit sind besonders hervorzuheben:
2-Methyl-5-[2-(2-hydroxy-3-isopropylamino-propoxy)-styryl]-1,3,4-thiadiazol
2-Methyl-5-[2-(2-hydroxy-3-cyclopropylamino-propoxy)-styryl]-1,3,4-thiadiazol
2-Methyl-5-[2-(2-hydroxy-3-tert.-butylamino-propoxy)-styryl]-1,3,4-thiadiazol
2-Methyl-5-[2-(2-hydroxy-3-(1-butin-3-ylamino)-propoxy)-styryl]-1,3,4-thiadiazol
vorliegende Erfindung wird durch die nachfolgenden Ausführungsbeispiele näher erläutert.

I. Herstellung von Ausgangsverbindungen

Verbindung 1

2-Methyl-5-(2-hydroxy-styryl)-1,3,4-thiadiazol

570 g (5 Mol) 2,5-Dimethyl-1,3,4-thiadiazol und 275 g (2,5 Mol) Salicylaldehyd werden gemischt und unter Durchleiten von Stickstoff langsam auf 150 °C erhitzt. Das Gemisch wird 30 h bei 150 °C gehalten, dann abgekühlt und nach Abdestillieren von überschüssigem 2,5-Dimethyl-1,3,4-thiadiazol aus Methylglykol umkristallisiert. Man erhält 304 g gelbe Kristalle (56 % d.Th.) vom Fp. 253 bis 254 °C.

$C_{11}H_{10}N_2OS$ (218)

| | | | |
|---|---|---|---|
| ber. | C 60,6 | H 4,6 | N 12,8 |
| gef. | C 59,8 | H 4,6 | N 12,4 |

Verbindung 2

2-Methyl-5-[2-(2,3-epoxypropoxy)-styryl]-1,3,4-thiadiazol

3,72 g (0,085 Mol) 55 %-iges Natriumhydrid in Paraffinöl werden in 150 ml abs. Tetrahydrofuran suspendiert und anschließend tropfenweise mit 18,6 g (0,085 Mol) 2-(2-Hydroxystyryl)-5-methyl-1,3,4-thiadiazol in 200 ml abs. Hexamethylphosphortriamid bei 0 bis 3 °C innerhalb von 1,5 h versetzt. Bei Raumtemperatur wird 1 h nachgerührt, anschließend werden 11,7 g (0,085 Mol) Dibromhydrin zugetropft. Man läßt 16 h bei Raumtemperatur stehen. Die Lösung wird auf 1,5 l Eiswasser und 0,5 l gesättigte Natriumchloridlösung gegeben. Der angefallene Feststoff wird abgesaugt und aus Aceton umkristallisiert. Man erhält 11,8 g (51 % d.Th.) gelbe Kristalle Fp. 134 bis 135 °C

$C_{14}H_{14}N_2O_2S$ (274)

| | | | | | |
|---|---|---|---|---|---|
| ber. | C 61,3 | H 5,1 | O 11,7 | S 11,7 | N 10,2 |
| gef. | C 61,3 | H 5,4 | O 13,5 | S 10,5 | N 8,4 |

Verbindung 3

2-Methyl-5-[2-(2-hydroxy-3-chlor-propoxy)-styryl]-1,3,4-thiadiazol

1 g 2-Methyl-5-[2-(2,3-epoxypropoxy)-styryl]-1,3,4-thiadiazol wird in einem Gemisch aus 100 ml Äthanol und 5 ml einer 3 normalen ätherischen Chlorwasserstoff-Lösung 12 Stunden stehengelassen. Der gebildete Niederschlag wird abgesaugt, mit Äther neutral gewaschen und mit Chloroform über Kieselgel chromatographiert. Die Produkteluate erbringen nach dem Eindampfen zur Trockene spektroskopisch reines 2-Methyl-5-[2-(2-hydroxy-3-chlor-propoxy)-styryl]-1,3,4-thiadiazol vom Schmp. 168 bis 170 °C.

1H-NMR-Spektrum ($CDCl_3$, TMS intern)

= 2,5-3,3 (m, 6H), 4,8 (s, 1H), 5,5-6,0 (m, 3H und OH), 6,1-6,3 (m, 2H), 7,3 (s, 3H)

II. Herstellung von erfindungsgemäßen Verbindungen

Beispiel 1

2-Methyl-5-[2-(2-hydroxy-3-isopropylamino-propoxy)-styryl]-1,3,4-thiadiazol

7 g (0,025 Mol) 2-Methyl-5-[2-(2,3-epoxy-propoxy)-styryl]-1,3,4-thiadiazol und 2,9 g (0,05 Mol) Isopropylamin werden in 100 ml Isopropanol zusammengegeben und für 7 Std. zum Rückfluß erhitzt. Nach dem Abkühlen wird das Lösungsmittel abdestilliert und der verbleibende Rückstand in Toluol umkristallisiert.

5,1 g (61 % d. Th.), Fp. 156 bis 157°

$C_{17}H_{23}O_2N_3S$ (333)

| | | | |
|---|---|---|---|
| ber. | C 61,2 | H 7,0 | N 12,6 |
| gef. | C 60,8 | H 6,8 | N 12,1 |

Beispiel 2

2-Methyl-5-[2-(2-hydroxy-3-cyclopropylamino-propoxy)-styryl]-1,3,4-thiadiazol

3,8 g (0,014 Mol) 2-Methyl-5-[2-(2,3-epoxy-propoxy)-styryl]-1,3,4-thiadiazol und 1,0 g (0,018 Mol) Cyclopropylamin werden analog Beispiel 1 umgesetzt. Man erhält aus Toluol 2,2 g (48 % d. Th.) gelbe

Kristalle, Fp. 144 bis 145 °C

$C_{17}H_{21}N_3O_2S$ (331)

| | | | |
|---|---|---|---|
| ber. | C 61,6 | H 6,4 | N 12,7 |
| gef. | C 60,8 | H 6,1 | N 12,3 |

Beispiel 3

2-Methyl-5-[2-(2-hydroxy-3-tert.-butylamino-propoxy)-styryl]-1,3,4-thiadiazol

7 g (0,025 Mol) 2-Methyl-5-[2-(2,3-epoxy-propoxy)-styryl]-1,3,4-thiadiazol und 2,1 g (0,028 Mol) tert.-Butylamin werden analog Beispiel 1 umgesetzt. Man erhält aus Toluol 3,8 g (44 % d. Th.) gelbe Kristalle. Fp. 110 bis 112 °C

$C_{18}H_{25}N_3O_2S$

| | | | |
|---|---|---|---|
| ber. | C 62,2 | H 7,2 | N 12,1 |
| gef. | C 62,3 | H 7,3 | N 11,6 |

Beispiel 4

2-Methyl-5-[2-(2-hydroxy-3-(butyl-2-amino)-propoxy)-styryl]-1,3,4-thiadiazol

Aus 6 g (0,022 Mol) 2-Methyl-5-[2-(2,3-epoxy-propoxy)-styryl]-1,3,4-thiadiazol und 1,6 g (0,022 Mol) 2-Butylamin werden analog Beispiel 1 3,5 g (45 % d. Th.) gelbe Kristalle erhalten. Fp. 135 bis 136 °C.

$C_{18}H_{25}N_3O_2S$ (347)

| | | | |
|---|---|---|---|
| ber. | C 62,2 | H 7,2 | N 12,1 |
| gef. | C 62,5 | H 7,1 | N 11,7 |

Beispiel 5

2-Methyl-5-[2-(2-hydroxy-3-(pentyl-2-amino)-propoxy)-styryl]-1,3,4-thiadiazol

6 g (0,022 Mol) 2-Methyl-5-[2-(2,3-epoxy-propoxy)-styryl]-1,3,4-thiadiazol und 1,9 g (0,022 Mol) 2-Amino-pentan werden analog Beispiel 1 umgesetzt. Man erhält 2,6 g (33 % d. Th.) gelbe Kristalle. Fp. 112 bis 113 °C.

$C_{19}H_{27}N_3O_2S$ (362)

| | | | |
|---|---|---|---|
| ber. | C 61,3 | H 7,5 | N 11,6 |
| gef. | C 63,3 | H 7,2 | N 11,7 |

Beispiel 6

2-Methyl-5-[2-(2-hydroxy-3-(1-butin-3-ylamino)-propoxy)styryl]-1,3,4-thiadiazol

6 g (0,022 Mol) 2-Methyl-5-[2-(2,3-epoxy-propoxy)-styryl]-1,3,4-thiadiazol und 1,5 g (0,022 Mol) 1-Butinylamin-3 werden analog Beispiel 1 umgesetzt. Die Reaktionslösung wird vom unlöslichen Rückstand abfiltriert und am Rotationsverdampfer eingeengt. Es verbleiten 6,2 g Öl, die an Kieselgel 60 (0,062 bis 0,200 mm) der Firma Merck chromatographisch gereinigt werden. Elutionsmittel Chloroform : Methanol 4 : 1. Das gereinigte Öl kann mit Isopropanol-Äther zur Kristallisation gebracht werden. 2,1 g (28 % d. Th.). Fp. 143 bis 145 °C.

$C_{18}H_{21}N_3O_2S$ (343)

| | | | |
|---|---|---|---|
| ber. | C 62,9 | H 6,2 | N 12,1 |
| gef. | C 61,6 | H 6,2 | N 12,3 |

Beispiel 7

2-Methyl-5-[2-(2-hydroxy-3-(3-methyl-1-butin-3-ylamino)-propoxy)-styryl]-1,3,4-thiadiazol

6 g (0,022 Mol) 2-Methyl-5-[2-(2,3-epoxy-propoxy)-styryl]-1,3,4-thiadiazol und 1,8 g (0,022 Mol) 3-Methyl-3-amino-butin-1 wurden analog Beispiel 7 umgesetzt. Man erhält 5,8 g gelbes Öl, das in 100 ml Isopropanol gelöst wird. Man versetzt mit ätherischer Salzsäure bis zur sauren Reaktion und gibt noch 100 ml Äther dazu. Der Rückstand wird abgesaugt, mit Äther gewaschen und getrocknet. 4,3 g (42 % d.Th.). Fp. 132 bis 133 °C.

$C_{19}H_{23}N_3O_2S \cdot 2\ HCl \cdot 2\ H_2O$ (466)

| | | | | |
|---|---|---|---|---|
| ber. | C 50,8 | H 6,0 | N 9,3 | Cl 15,8 |
| gef. | C 50,0 | H 5,8 | N 8,5 | Cl 14,4 |

## Beispiel 8

2-Methyl-5-[2-(2-hydroxy-3-(3-äthyl-1-pentin-3-ylamino)-propoxy)-styryl]-1,3,4-thiadiazol

Aus 7 g (0,025 Mol) 2-Methyl-5-[2-(2,3-epoxy-propoxy)-styryl]-1,3,4-thiadiazol und 2,8 g (0,025 Mol) 3-Äthyl-3-amino-pentin-1 werden analog Beispiel 8 3,7 g (32 % d.Th.) des Hydrochlorids vom Fp. 157 bis 159 °C erhalten.

$C_{21}H_{27}N_3O_2S \cdot 1{,}5\ HCl \cdot 1{,}5\ H_2O$ (467)

| | | | | |
|---|---|---|---|---|
| ber. | C 53,9 | H 6,7 | N 8,9 | Cl 11,5 |
| gef. | C 53,4 | H 6,5 | N 8,9 | Cl 11,8 |

## Beispiel 9

2-Methyl-5-[2-(2-hydroxy-3-tert.-butylamino-propoxy)-styryl]-1,3,4-thiadiazol

In einem Autoklaven werden 1,6 g 2-Methyl-5-[2-(2-hydroxy-3-chlor-propoxy)-styryl]-1,3,4-thiadiazol und 10 ml tert.-Butylamin in 50 ml Dioxan 10 Stunden auf 100 °C erhitzt. Nach dem Abdestillieren der flüchtigen Anteile im Vakuum wird der hochviskose Rohaustrag mit Chloroform über eine Kieselgeltrockensäule chromatographiert. Der Eindampfrückstand der Produkteluate ergibt 2-Methyl-5-[2-(2-hydroxy-3-tert.-butylamino-propoxy)-styryl]-1,3,4-thiadiazol. Die isolierte Verbindung ist mit der nach Beispiel 3 erhaltenen Verbindung identisch.

Auch die Verbindungen der Beispiele 1, 2 und 4 bis 8 werden in gleicher Weise erhalten, wenn man 2-Methyl-5-[2-(2-hydroxy-3-chlor-propoxy)-styryl]-1,3,4-thiadiazol mit dem entsprechenden Amin umsetzt.

Formulierungsbeispiele, die in üblicher Weise hergestellt werden :

1. Tabletten:

a) 

| | |
|---|---|
| Ein Wirkstoff der Formel I | 5 mg |
| Lactose | 200 mg |
| Methylcellulose | 15 mg |
| Maisstärke | 50 mg |
| Talkum | 11 mg |
| Magnesiumstearat | 4 mg |
| | 285 mg |

b) 

| | |
|---|---|
| Ein Wirkstoff der Formel I | 20 mg |
| Lactose | 178 mg |
| Avicel | 80 mg |
| Polywachs 6 000 | 20 mg |
| Magnesiumstearat | 2 mg |
| | 300 mg |

c) 

| | |
|---|---|
| Ein Wirkstoff der Formel I | 50 mg |
| Polyvinylpyrrolidon (mittl. M.G. 25 000) | 170 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |
| | 280 mg |

Der Wirkstoff wird mit Polyvinylpyrrolidon in 10 %-iger wäßriger Lösung befeuchtet, durch ein Sieb mit der lichten Maschenweite 1,0 mm getrieben und bei 50 °C getrocknet. Dieses Granulat wird mit Polyäthylenglykol (mittl. M.G. 4 000), Hydroxypropylmethylcellulose, Talkum und Magnesiumstearat vermischt und zu Tabletten a 280 mg verpreßt.

2. Beispiel für Dragees :

| | |
|---|---|
| Ein Wirkstoff der Formel I | 60 mg |
| Lactose | 90 mg |
| Maisstärke | 60 mg |
| Polyvinylpyrrolidon | 6 mg |
| Magnesiumstearat | 1 mg |
| | 217 mg |

Die Mischung der Wirkstoffsubstanz mit Lactose und Maisstärke wird mit einer 8 %-igen wäßrigen Lösung des Polyvinylpyrrolidons durch Sieb 1,5 mm granuliert, bei 50 °C getrocknet und nochmals durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

3. Kapselformulierung :

| | |
|---|---|
| Ein Wirkstoff der Formel I | 5,0 mg |
| Magnesiumstearat | 2,0 mg |
| Milchzucker | 19,3 mg |

4. Injektionslösung :

| | |
|---|---|
| Ein Wirkstoff der Formel I | 10 mg |
| Natriumchlorid | 9 mg |
| destilliertes Wasser, q.s. auf 1,0 ml | |

**Ansprüche**

1. Verbindungen der allgemeinen Formel (I),

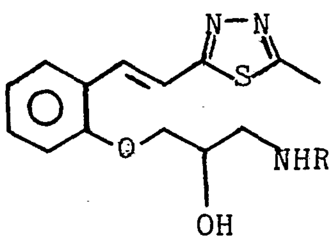

(I)

in denen R einen am zum Stickstoffatom α-ständigen Kohlenstoffatom verzweigten Alkylrest mit 3 bis 6 C-Atomen, einen Alkenyl- oder Alkinylrest mit je 2 bis 8 C-Atomen oder Cyclopropyl bedeutet, und ihre Säureadditionssalze.

2. 2-Methyl-5-[2-(2-hydroxy-3-isopropylamino-propoxy)-styryl]-1,3,4-thiadiazol.
3. 2-Methyl-5-[2-(2-hydroxy-3-cyclopropylamino-propoxy)-styryl]-1,3,4-thiadiazol.
4. 2-Methyl-5-[2-(2-hydroxy-3-tert.-butylamino-propoxy)-styryl]-1,3,4-thiadiazol.
5. 2-Methyl-5-[2-(2-hydroxy-3-(1-butin-3-ylamino)-propoxy)-styryl]-1,3,4-thiadiazol.
6. Verfahren zur Herstellung von Verbindungen der allgemeinen Formel (I) nach Anspruch 1, dadurch gekennzeichnet, daß man ein 2-Methyl-5-styryl-1,3,4-thiadiazol der allgemeinen Formel (II)

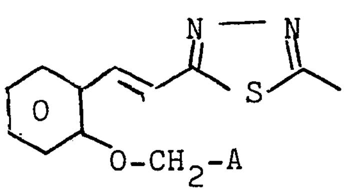

(II)

in der A den Rest

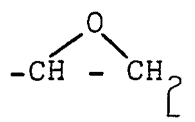 oder 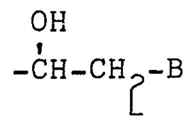

wobei B für eine nukleofuge Abgangsgruppe steht, bedeutet, mit einem Amin der allgemeinen Formel

$$H_2N—R$$

in der R die für Formel I angegebenen Bedeutungen hat, zweckmäßigerweise in einem Lösungmittel und ggf. in Gegenwart eines säurebindenden Mittels bei Temperaturen von 10 bis 120 °C umsetzt und die erhaltene Verbindung gegebenenfalls in das Säureadditionssalz einer physiologisch verträglichen Säure überführt.

7. Therapeutische Mittel, gekennzeichnet durch einen Gehalt an einer Verbindung der Formel I oder ihres physiologisch verträglichen Säureadditionssalzes nach Anspruch 1 als Wirkstoff neben üblichen Trägerstoffen und Verdünnungsmitteln.

8. Therapeutisches Mittel nach Anspruch 7, gekennzeichnet durch einen Gehalt an 2-Methyl-5-[2-(2-hydroxy-3-isopropylamino-propoxy)-styryl]-1,3,4-thiadiazol oder dessen physiologisch verträglichen Säureadditionssalzes als Wirkstoff neben üblichen Trägerstoffen und Verdünnungsmitteln.

9. Therapeutisches Mittel nach Anspruch 7, gekennzeichnet durch einen Gehalt an 2-Methyl-5-[2-(2-hydroxy-3-cyclopropylamino-propoxy)-styryl]-1,3,4-thiadiazol oder dessen physiologisch verträglichen Säureadditionssalzes als Wirkstoff neben üblichen Trägerstoffen und Verdünnungsmitteln.

10. Therapeutisches Mittel nach Anspruch 7, gekennzeichnet durch einen Gehalt an 2-Methyl-5-[2-(2-hydroxy-3-tert.-butylamino-propoxy)-styryl]-1,3,4-thiadiazol oder dessen physiologisch verträglichen Säureadditionssalzes als Wirkstoff neben üblichen Trägerstoffen und Verdünnungsmitteln.

11. Therapeutisches Mittel nach Anspruch 7, gekennzeichnet durch einen Gehalt an 2-Methyl-5-[2-(2-hydroxy-3-(1-butin-3-ylamino)-propoxy)-styryl]-1,3,4-thiadiazol oder dessen physiologisch verträglichen Säureadditionssalzes als Wirkstoff neben üblichen Trägerstoffen und Verdünnungsmitteln.

**Claims**

1. Compounds of the general formula (I)

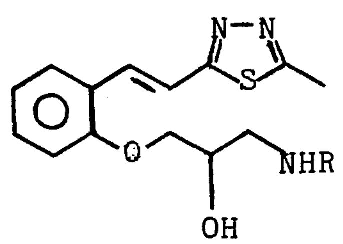

(I)

where R is alkyl of 3 to 6 carbon atoms which is branched at the carbon in the α-position to the nitrogen, alkenyl or alkynyl of 2 to 8 carbon atoms or cyclopropyl, and their acid addition salts.

2. 2-Methyl-5-[2-(2-hydroxy-3-isopropylamino-propoxy)-styryl]-1,3,4-thiadiazole.

3. 2-Methyl-5-[2-(2-hydroxy-3-cyclopropylamino-propoxy)-styryl]-1,3,4-thiadiazole.

4. 2-Methyl-5-[2-(2-hydroxy-3-tert.-butylamino-propoxy)-styryl]-1,3,4-thiadiazole.

5. 2-Methyl-5-[2-(2-hydroxy-3-(but-1-ynyl-3-amino)-propoxy)-styryl]-1,3,4-thiadiazole.

6. A process for the preparation of compounds of the general formula (I) as claimed in claim 1, characterized in that a 2-methyl-5-styryl-1,3,4-thiadiazole of the general formula (II)

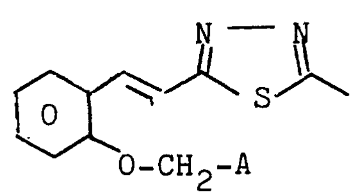

(II)

where A is

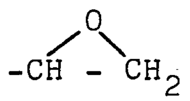

or

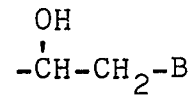

B being a nucleofugic leaving group, is reacted with an amine of the general formula

$H_2N—R$

where R has the meanings given for formula I, advantageously in a solvent, and in the presence or absence of an acid-binding agent, at from 10 to 120 °C, and, if required, the compound obtained is converted into the addition salt with a physiologically acceptable acid.

7. A therapeutic agent characterized by a content of a compound of the formula I, or an addition salt thereof with a physiologically acceptable acid, as claimed in claim 1, as the active ingredient, in addition to conventional excipients and diluents.

8. A therapeutic agent as claimed in claim 7, characterized by a content of 2-methyl-5-[2-(2-hydroxy-3-isopropylamino-propoxy)-styryl]-1,3,4-thiadiazole or an addition salt thereof with a physiologically acceptable acid, as the active ingredient, in addition to conventional excipients and diluents.

9. A therapeutic agent as claimed in claim 7, characterized by a content of 2-methyl-5-[2-(2-hydroxy-3-cyclopropylamino-propoxy)-styryl]-1,3,4-thiadiazole or an addition salt thereof with a physiologically acceptable acid, as the active ingredient, in addition to conventional excipients and diluents.

10. A therapeutic agent as claimed in claim 7, characterized by a content of 2-methyl-5-[2-(2-hydroxy-3-tert.-butylamino-propoxy)-styryl]-1,3,4-thiadiazole or an addition salt thereof with a physiologically acceptable acid, as the active ingredient, in addition to conventional excipients and diluents.

11. A therapeutic agent as claimed in claim 7, characterized by a content of 2-methyl-5-[2-(2-hydroxy-3-(but-1-ynyl-3-amino)-propoxy)-styryl]-1,3,4-thiadiazole or an addition salt thereof with a physiologically acceptable acid, as the active ingredient, in addition to conventional excipients and diluents.

**Revendications**

1. Composés de la formule générale I

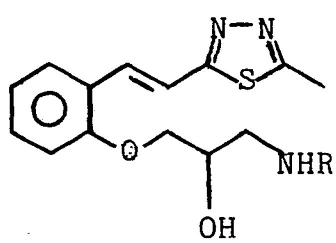

(I)

dans laquelle R désigne un radical alcoyle en $C_3$ à $C_6$, ramifié à l'atome de carbone en position α par rapport à l'atome d'azote, un groupe alcényle ou alcynyle en $C_2$ à $C_8$ ou un groupe cyclopropyle, et leurs sels d'addition d'acides.

2. Le 2-méthyl-5-[2-(2-hydroxy-3-isopropylamino-propoxy)-styryl]-1,3,4-thiadiazole.

3. Le 2-méthyl-5-[2-(2-hydroxy-3-cyclopropylamino-propoxy)-styryl]-1,3,4-thiadiazole.

4. Le 2-méthyl-5-[2-(2-hydroxy-3-tertiobutylamino-propoxy)-styryl]-1,3,4-thiadiazole.

5. Le 2-méthyl-5-[2-(2-hydroxy-3-(1-butyne-3-yl-amino)-propoxy)-styryl]-1,3,4-thiadiazole.

6. Procédé de préparation de composés de la formule générale I suivant la revendication 1, caractérisé en ce que l'on fait réagir un 2-méthyl-5-styryl-1,3,4-thiadiazole de la formule générale II

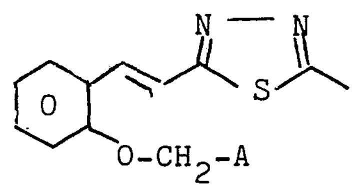

(II)

dans laquelle A représente un groupe

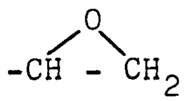

ou

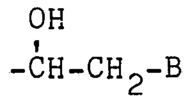

,

B désignant un groupe nucléofuge clivable, avec une amine de la formule générale

$$H_2N—R$$

dans laquelle R possède les significations définies pour la formule I, à une température de 10 à 120 °C, généralement dans un solvant et éventuellement en présence d'un agent fixant l'acide, le composé formé étant le cas échéant transformé en sel d'addition d'un acide physiologiquement acceptable.

7. Composition thérapeutique, caractérisée par une teneur en un composé de la formule I suivant la revendication 1 ou en un sel d'addition d'un acide physiologiquement acceptable d'un tel composé comme principe actif à côté de véhicules et diluants usuels.

8. Composition thérapeutique suivant la revendication 7, caractérisée par une teneur en 2-méthyl-5-[2-(2-hydroxy-3-isopropylamino-propoxy)-styryl]-1,3,4-thiadiazole ou en un sel d'addition d'un acide physiologiquement acceptable de celui-ci comme principe actif à côté de véhicules et diluants usuels.

9. Composition thérapeutique suivant la revendication 7, caractérisée par une teneur en 2-méthyl-5-[2-(2-hydroxy-3-cyclopropylamino-propoxy)-styryl]-1,3,4-thiadiazole ou en un sel d'addition d'un acide physiologiquement acceptable de celui-ci comme principe actif à côté de véhicules et diluants usuels.

10. Composition thérapeutique suivant la revendication 7, caractérisée par une teneur en 2-méthyl-5-[2-(2-hydroxy-3-tertiobutylamino-propoxy)-styryl]-1,3,4-thiadiazole ou en un sel d'addition d'un acide physiologiquement acceptable de celui-ci comme principe actif à côté de véhicules et diluants usuels.

11. Composition thérapeutique suivant la revendication 7, caractérisée par une teneur en 2-méthyl-5-[2-(2-hydroxy-3-(1-butyne-3-yl-amino)-propoxy)-styryl]-1,3,4-thiadiazole ou en un sel d'addition d'un acide physiologiquement acceptable de celui-ci comme principe actif à côté de véhicules et diluants usuels.